# EUROPÄISCHE PATENTANMELDUNG

(11) **EP 3 135 386 A1**
(43) Veröffentlichungstag der Anmeldung: **01.03.2017**
(21) Anmeldenummer: 16020105.9
(22) Anmeldetag: 24.02.2005
(51) Int. Cl.: B05B 11/00, A61M 15/00, A61M 1/00

(54) **ZERSTÄUBER**

(30) Priorität: 24.02.2004 DE 102004009435
(62) Teilanmeldung aus: 05715510.3
(71) Anmelder: Boehringer Ingelheim International GmbH, 55216 Ingelheim am Rhein (DE)
(72) Erfinder: Wuttke, Gilbert, 55216 Ingelheim am Rhein (DE); Noehl, Klaus, 55216 Ingelheim am Rhein (DE); Geser, Johannes, 55216 Ingelheim am Rhein (DE); Kaulmann, Stefan, 55216 Ingelheim am Rhein (DE); Kunze, Herbert, 55216 Ingelheim am Rhein (DE); Fiol, Andreas, 55216 Ingelheim am Rhein (DE); Zierenberg, Bernd, 55216 Ingelheim am Rhein (DE)
(74) Vertreter: Von Rohr Patentanwälte Partnerschaft mbB

(57) **Zusammenfassung**

Es wird ein Zerstäuber mit einem einsetzbarem Behälter und einer Überwachungs-einrichtung zur Zählung von Betätigungen des Zerstäubers vorgeschlagen. Die Überwachungseinrichtung ist in einem lösbaren Gehäuseteil angeordnet und erfasst unmittelbar Bewegungen des Behälters während eines Zerstäubungsvor-gangs, einen Zuluftstrom im Bereich eines Mundstücks und/oder die Erzeugung von Aerosol, um dies als tatsächlichen Austrag von Fluid zu detektieren und Betätigung des Zerstäubers zu zählen, wobei vorzugsweise zusätzlich der jeweilige Zeitpunkt der Betätigung des Zerstäubers erfasst und gespeichert wird. So werden eine verbesserte Überwachung und Benutzerführung ermöglicht.

## Beschreibung

Die vorliegende Erfindung betrifft einen Zerstäuber gemäß dem Oberbegriff des Anspruchs 1.

Ausgangspunkt der vorliegenden Erfindung ist ein unter dem Handelsnamen "Respimat" von der Boehringer Ingelheim KG angebotener Zerstäuber in Form eines Inhalators, wie im Grundprinzip in der WO 91/14468 A1 und in konkreter Ausgestaltung in der WO 97/12687 A1 (Fig. 6a, 6b) sowie in Fig. 1 und 2 der anliegenden Zeichnung dargestellt. Der Zerstäuber weist als Reservoir für ein zu zerstäubendes Fluid einen einsetzbaren Behälter mit dem Fluid und einen Druckerzeuger mit einer Antriebsfeder zur Förderung und Zerstäubung des Fluids auf. Durch Drehen eines Betätigungsteils in Form eines Gehäuseunterteils des Zerstäubers ist die Antriebsfeder spannbar und Fluid in eine Druckkammer des Druckerzeugers saugbar. Nach manueller Betätigung eines Sperrelements wird das Fluid in der Druckkammer von der Antriebsfeder unter Druck gesetzt und zerstäubt, also unter Bildung eines Aerosols ausgegeben. Beim Spannen einerseits und der dann folgenden Zerstäubung andererseits führt der Behälter jeweils eine Hubbewegung aus. Der Zerstäuber weist eine mechanische Überwachungseinrichtung auf, die zur Zählung von Betätigungen des Zerstäubers das Drehen des Betätigungsteils erfasst. Der bekannte Zerstäuber arbeitet ausschließlich mechanisch, d. h. ohne Treibgas und ohne Elektrik.

Zur Vervollständigung der Offenbarung der vorliegenden Patentanmeldung wird vorsorglich auf den kompletten Offenbarungsgehalt sowohl der WO 91/14468 A1 als auch der WO 97/12687 A1 verwiesen. Generell bezieht sich die dortige Offenbarung bevorzugt auf einen Zerstäuber mit einem Federdruck von 5 bis 60 MPa, bevorzugt 10 bis 50 MPa auf das Fluid, mit Volumina pro Hub von 10 bis 50 µl, bevorzugt 10 bis 20 µl, ganz bevorzugt etwa 15 µl pro Hub, Teilchengrößen von bis zu 20 µm, bevorzugt 3 bis 10 µm. Ferner bezieht sich die dortige Offenbarung bevorzugt auf einen Zerstäuber mit zylinderähnlicher Form und einer Größe von etwa 9 cm bis etwa 15 cm in der Länge und etwa 2 cm bis etwa 5 cm in der Breite sowie von einer Düsen-Strahlfächerung von 20° bis 160°, bevorzugt von 80° bis 100°. Derartige Werte gelten auch für den Zerstäuber nach der Lehre der Erfindung als besonders bevorzugte Werte.

Bekannt ist ferner eine Vorrichtung zum Erfassen der Betätigung eines Spenders, wobei ein Austragsförderer durch eine Hubbewegung zwischen einem Betätigungselement und einem Medienbehälter betätigt wird und im Betätigungselement ein Schalter zur Erfassung einer Betätigung und Erzeugung eines elektrischen Zählsignals angeordnet ist (DE 100 65 160 A1). Der Schalter wird hier bei der linearen Hubbewegung nicht unmittelbar vom Behälter, sondern von einer Befestigungsschraube der Vorrichtung betätigt, so dass bei einer Betätigung auch bei nicht eingesetztem Behälter ein Zählsignal erzeugt wird.

Die DE 100 61 723 C2 offenbart ein mechanisches Zählwerk zum Zählen dosierter Abgaben flüssiger, pastöser oder fester Produkte, insbesondere Medikamente, aus einem Vorratsbehälter, insbesondere einem Aerosolbehälter. Vorzugsweise wird eine lineare Bewegung des Aerosolbehälters gezählt.

Bekannt ist des weiteren eine Austragsteuerung für einen Medien-Spender (DE 198 07 921 A1). Diese Steuerung umfasst einen Speicher und eine Intervall-Schaltung, welche einen möglichen Betätigungshub nur zu bestimmten Zeiten freigibt und sonst sperrt. Der Speicher ist mit einem Computer so programmierbar, dass die Sperre nur zu bestimmten Zeiten freigegeben wird, wofür ein Programm ein Zeitschaltglied umfasst. Eine Anzeige zeigt dem Benutzer an, wann eine Mediendosis appliziert werden soll und wann nicht. Der Speicher kann diese Anwendungen erfassen, wonach sie jederzeit mit einem Computer auf einem Bildschirm erkennbar gemacht werden können. Zur Programmierung und Abfrage des Speichers bzw. zur Aufladung eines Energiespeichers kann die Steuerung einen von außen zugänglichen elektrischen Anschluss für einen entsprechenden Stecker aufweisen.

Der vorliegenden Erfindung liegt die Aufgabe zugrunde, einen Zerstäuber der eingangs genannten Art mit einer verbesserten Überwachungseinrichtung anzugeben, insbesondere wobei eine gesteigerte Benutzungssicherheit und ggf. eine weitergehende Information des Benutzers und/oder eine Benutzerüberwachung ermöglicht wird bzw. werden.

Die obige Aufgabe wird durch einen Zerstäuber gemäß Anspruch 1 gelöst. Vorteilhafte Weiterbildungen sind Gegenstand der Unteransprüche.

Gemäß einem ersten Aspekt der vorliegenden Erfindung ist die Überwachungseinrichtung an einem lösbaren Gehäuseteil des Zerstäubers angeordnet, insbesondere fest mit diesem verbunden, vorzugsweise in dieses eingegossen. Dies gestattet ein einfaches Lösen der Überwachungseinrichtung zusammen mit dem Gehäuseteil vom Zerstäuber, so dass die Überwachungseinrichtung sehr einfach - getrennt bzw. unabhängig vom Zerstäuber - eingeschaltet, programmiert, initialisiert und/oder ausgelesen werden kann und/oder so dass sehr einfach ein vollständiger Austausch der Überwachungseinrichtung zusammen mit dem Gehäuseteil oder eine Nachrüstung eines Zerstäubers mit einer Überwachungseinrichtung bei entsprechender Kompatibilität des Gehäuseteils ermöglicht wird.

Ein weiterer, auch unabhängig realisierbarer Aspekt der vorliegenden Erfindung liegt darin, dass ein tatsächlicher Austrag von Fluid erfasst und insbesondere elektronisch als Betätigung des Zerstäubers gezählt wird. Dies gestattet eine verbesserte Überwachung sowie eine verbesserte Benutzungssicherheit und Benutzerführung.

Ein tatsächlicher Austrag wird vorzugsweise dadurch detektiert bzw. erfasst, indem unmittelbar eine Bewegung, vorzugsweise ein Hub, des Behälters erfasst wird, dass ein Aufnahmesensor einen durch das Inhalieren hervorgerufenen Zuluftstrom detektiert und/oder dass ein Spraysensor eine Erzeugung von zerstäubtem Fluid bzw. Aerosol, insbesondere im Bereich eines Mundstücks, detektiert. Dementsprechend ist mit wesentlich größerer Sicherheit feststellbar, ob tatsächlich ein Austrag von Fluid bzw. tatsächlich ein Inhalieren erfolgt ist. Vorzugsweise erfolgt die Überwachung nicht nur qualitativ, sondern auch quantitativ.

Gemäß einer Variante ist vorgesehen, dass die Überwachungseinrichtung mittels des Aufnahmesensors ein ausreichend starkes und/oder andauerndes Inhalieren des von dem Zerstäuber zerstäubten Fluids erfasst und insbesondere speichert und/oder als (erfolgreiche) Betätigung des Zerstäubers bzw. Einnahme des Fluids zählt. Dies ist einer sichereren Bedienung und besseren Überwachung zuträglich.

Besonders bevorzugt ist die Überwachungseinrichtung des Zerstäubers mit einem Zeitgeber und einem Speicher versehen, so dass Anzahl und Zeitpunkt von Betätigungen des Zerstäubers erfassbar und speicherbar sind und/oder so dass eine wiederholte Betätigung innerhalb einer vorbestimmte Mindestzeitdauer blockierbar und/oder so dass vorzugsweise nach Ablauf einer vorbestimmten Höchstzeitdauer ein Erinnerungssignal vorzugsweise zur erneuten Betätigung ausgebbar oder anzeigbar ist.

Bei Erfassung und Speicherung der Anzahl und Zeitpunkte des tatsächlichen Austrags von Fluid ist eine kontinuierliche Überwachung, beispielsweise vom Arzt oder bei klinischen Studien, möglich. Durch Abfrage der Überwachungseinrichtung bzw. des Speichers ist damit feststellbar, wann Austräge erfolgten und ggf. welche Mengen vom Zerstäuber abgegeben wurden.

Eine Blockierung einer wiederholten Betätigung des Zerstäubers innerhalb einer vorbestimmten, vorzugsweise vorgebbaren und speicherbaren Mindestzeitdauer kann eine Überdosierung des Fluids, bei dem es sich ja vorzugsweise um ein hochwirksames Arzneimittel handelt, verhindern.

Durch Ausgabe eines Erinnerungssignals vorzugsweise zur erneuten Betätigung des Zerstäubers beispielsweise nach Ablauf einer vorbestimmten, vorzugsweise vorgebbaren und speicherbaren Höchstzeitdauer kann ein Benutzer daran erinnert werden, dass eine erneute Inhalation erfolgen sollte. So kann eine regelmäßige Inhalation, also Aufnahme des Fluids, unterstützt werden. Insbesondere kann das Erinnerungssignal die Zeit bis zur nächsten Inhalation bzw. Betätigung oder ggf. einer überfälligen Inhalation bzw. Betätigung anzeigen. Besonders bevorzugt ist das Erinnerungssignal ein Vorwarn- oder Alarmsignal insbesondere wie in der WO 03/092576 A2 offenbart, die hiermit vollumfänglich als ergänzende Offenbarung eingeführt wird.

Weitere Vorteile, Merkmale, Eigenschaften und Aspekte der vorliegenden Erfindung ergeben sich aus der folgenden Beschreibung bevorzugter Ausführungsformen anhand der Zeichnung. Es zeigt:
- Fig. 1: einen schematischen Schnitt eines bekannten Zerstäubers im ungespannten Zustand;
- Fig. 2: einen schematischen, um 90° gegenüber Fig. 1 gedrehten Schnitt des bekannten Zerstäubers im gespannten Zustand;
- Fig. 3: eine schematische Schnittdarstellung eines unteren Gehäuseteils eines vorschlagsgemäßen Zerstäubers mit integrierter Überwachungseinrichtung;
- Fig. 4: eine blockschaltbildartige Darstellung der Überwachungseinrichtung;
- Fig. 5: eine schematische Darstellung einer Anschlusseinrichtung für die Überwachungseinrichtung; und
- Fig. 6: einen schematischen Schnitt eines anderen vorschlagsgemäßen Zerstäubers mit einem Aufnahmesensor und einem Spraysensor.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

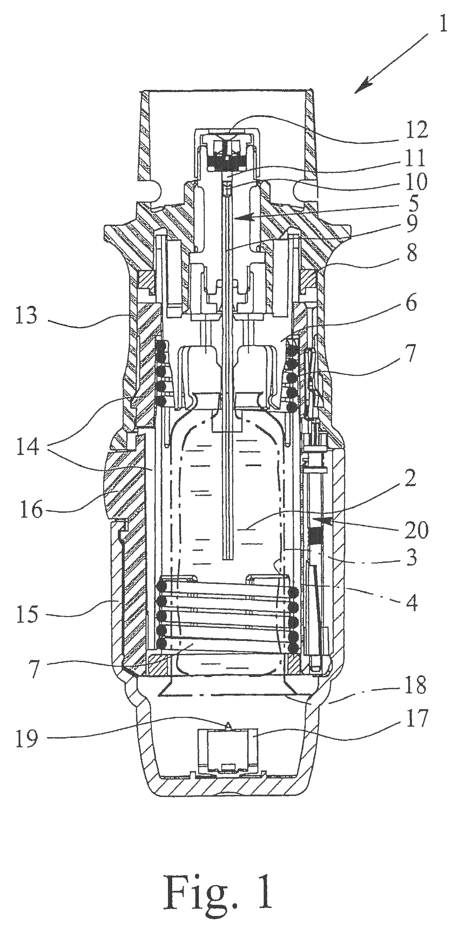

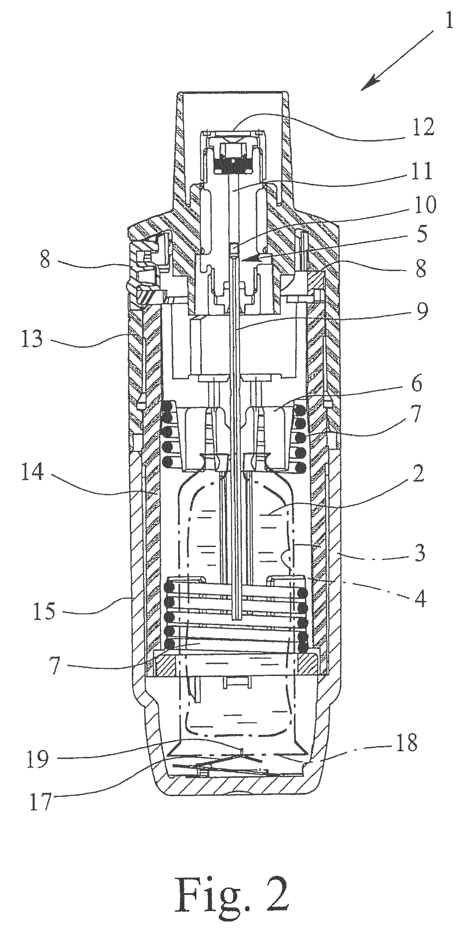

Fig. 1 und 2 zeigen einen bekannten Zerstäuber 1 zur Zerstäubung eines Fluids 2, insbesondere eines hochwirksamen Arzneimittels o.dgl., in einer schematischen Darstellung im ungespannten Zustand (Fig. 1) und gespannten Zustand (Fig. 2). Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, wird ein Aerosol gebildet, das von einem nicht dargestellten Benutzer eingeatmet bzw. inhaliert werden kann. Üblicherweise erfolgt das Inhalieren wenigstens einmal täglich, insbesondere mehrmals täglich, vorzugsweise in vorbestimmten Zeitabständen.

Der Zerstäuber 1 weist einen einsetzbaren und vorzugsweise wechselbaren Behälter 3 mit dem Fluid 2 auf, der ein Reservoir für das zu zerstäubende Fluid 2 bildet. Vorzugsweise enthält der Behälter 3 eine ausreichende Menge an Fluid 2 für eine mehrfache Anwendung, insbesondere für eine vorbestimmte Applikationszeit, wie einen Monat, oder für mindestens 50, vorzugsweise mindestens 100, Dosierungen bzw. Zerstäubungen.

Der Behälter 3 ist im wesentlichen zylindrisch bzw. kartuschenartig ausgebildet und von unten, nach Öffnen des Zerstäubers 1, in diesen einsetzbar und ggf. wechselbar. Er ist vorzugsweise starr ausgebildet, insbesondere wobei das Fluid 2 in einem Beutel 4 im Behälter 3 aufgenommen ist.

Der Zerstäuber 1 weist einen Druckerzeuger 5 zur Förderung und Zerstäubung des Fluids 2, insbesondere jeweils in einer vorbestimmten, ggf. einstellbaren Dosiermenge, auf. Der Druckerzeuger 5 weist eine Halterung 6 für den Behälter 3, eine zugeordnete Antriebsfeder 7 mit einem zur Entsperrung manuell betätigbaren Sperrelement 8, ein Förderrohr 9 mit einem Rückschlagventil 10, eine Druckkammer 11 und eine Austragsdüse 12 auf.

Beim axialen Spannen der Antriebsfeder 7 wird die Halterung 6 mit dem Behälter 3 und dem Förderrohr 9 bei den Darstellungen nach unten bewegt und Fluid 2 aus dem Behälter 3 in die Druckkammer 11 des Druckerzeugers 5 über das Rückschlagventil 10 gesaugt. Da die Austragsdüse 12 einen sehr geringen Strömungsquerschnitt hat und insbesondere als Kapillare ausgebildet ist, ergibt sich eine so starke Drosselwirkung, dass auch ohne Rückschlagventil an dieser Stelle ein Einsaugen von Luft sicher ausgeschlossen ist.

Beim anschließenden Entspannen nach Betätigung des Sperrelements 8 wird das Fluid 2 in der Druckkammer 11 von der das Förderrohr 9 wieder nach oben bewegenden Antriebsfeder 7 - also durch Federkraft - unter Druck gesetzt und über die Austragsdüse 12 ausgegeben, wobei es zerstäubt wird, insbesondere in Partikel im µm- oder nm-Bereich, vorzugsweise lungengängige Partikel mit etwa 5 µm. Die Förderung und Zerstäubung des Fluids 2 erfolgen vorzugsweise also rein mechanisch, insbesondere ohne Treibgas und ohne Elektrik.

Der Zerstäuber 1 weist ein Gehäuseoberteil 13 und ein demgegenüber drehbares Innenteil 14 auf, an dem ein Betätigungsteil 15 vorzugsweise mittels eines Halteelementes 16 lösbar befestigt, insbesondere aufgesteckt, ist. Zum Einsetzen und/oder Auswechseln des Behälters 3 ist das Betätigungsteil 15 vom Zerstäuber 1 lösbar.

Durch manuelles Drehen des Betätigungsteils 15 ist das Innenteil 14 relativ zum Gehäuseoberteil 13 drehbar, wodurch die Antriebsfeder 7 über ein nicht dargestelltes, auf die Halterung 6 wirkendes Getriebe in axialer Richtung spannbar ist. Beim Spannen wird der Behälter 3 axial nach unten bewegt, bis der Behälter 3 eine in Fig. 2 angedeutete Endlage im gespannten Zustand annimmt. Während des Zerstäubungsvorgangs wird der Behälter 3 von der Antriebsfeder 7 wieder in seine Ausgangslage zurückbewegt. Das axiale Bewegen des Behälters 3 beim Betätigen des Zerstäubers 1 wird nachfolgend auch als Hub des Behälters 3 bezeichnet.

Das Betätigungsteil 15 bildet vorzugsweise ein kappenartiges Gehäuseunterteil und um- bzw. übergreift einen unteren freien Endbereich des Behälters 3. Beim Spannen der Antriebsfeder 7 bewegt sich der Behälter 3 mit seinem Endbereich (weiter) in das Betätigungsteil 15 bzw. zu dessen stirnseitigem Ende hin, wobei eine axial wirkende, im Betätigungsteil 15 angeordnete Feder 17 am Behälterboden 18 zur Anlage kommt und mit einem Anstechelement 19 den Behälter 3 bei der erstmaligen Anlage zur Belüftung ansticht.

Der Zerstäuber 1 weist eine Überwachungseinrichtung 20 auf, die Betätigungen des Zerstäubers 1 zählt, indem sie ein Drehen des Innenteils 14 zum Gehäuseoberteil 13 erfasst. Die Überwachungseinrichtung 20 arbeitet rein mechanisch.

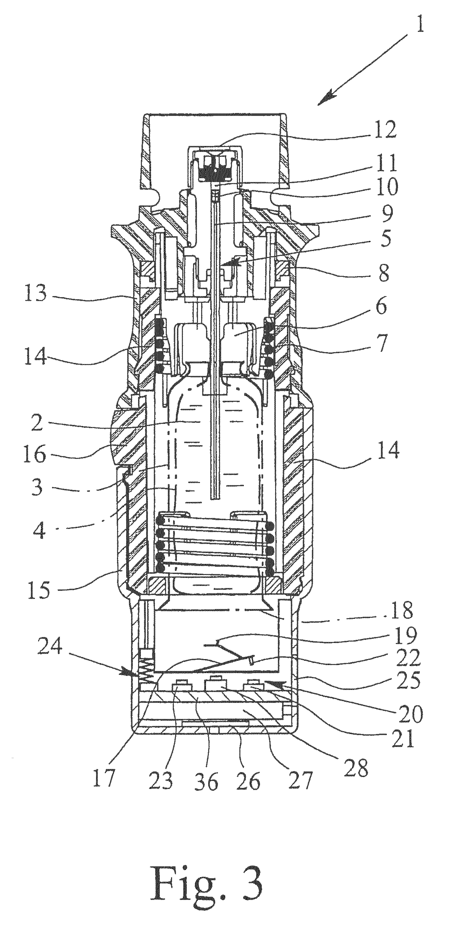

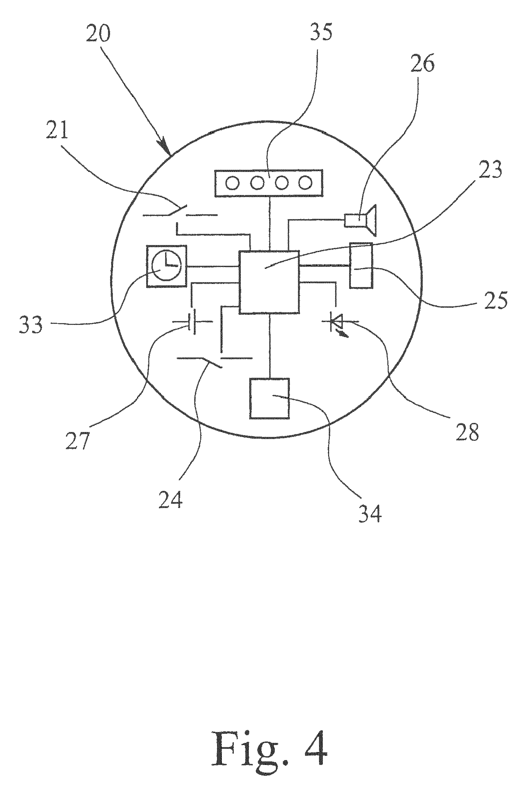

Nachfolgend werden der Aufbau und die Funktionsweise eines vorschlagsgemäßen Zerstäuber 1 mit einer modifizierten Überwachungseinrichtung 20 näher erläutert, wobei auf die Schnittansicht gemäß Fig. 3 und den blockschaltbildartigen Aufbau gemäß Fig. 4 Bezug genommen wird und im übrigen die Ausführungen zu Fig. 1 und 2 gelten.

Die Überwachungseinrichtung 20 ist vorzugsweise in ein lösbares und vorzugsweise auswechselbares Gehäuseteil des Zerstäubers 1, insbesondere in das Betätigungsteil 15 des Zerstäubers 1, eingebaut. Die Überwachungseinrichtung 20 ist vorzugsweise im Bereich des axialen Endes des Zerstäubers 1 bzw. des Betätigungsteils 15 angeordnet, insbesondere eingegossen.

Bei eingesetztem Behälter 3 ist die Überwachungseinrichtung 20 vorzugsweise benachbart zu dem Behälterboden 18 des Behälters 3 und/oder in Verlängerung der Bewegungs- bzw. Hubrichtung des Behälters 3 angeordnet.

Die Überwachungseinrichtung 20 erfasst als tatsächlichen Austrag von Fluid 2 vorzugsweise Bewegungen bzw. Hübe des Behälters 3 vorzugsweise mechanisch, optisch, elektrisch, induktiv, kapazitiv und/oder anderweit berührungslos. Insbesondere weist die Überwachungseinrichtung 20 gemäß dem Darstellungsbeispiel einen Mikroschalter 21 oder sonstigen Schalter, beispielsweise einen Näherungsschalter, induktiven Schalter, kapazitiven Schalter oder Reed-Kontakt, oder geeigneten Sensor auf.

Der hier konkret vorgesehene Mikroschalter 21 ist hier von einem Vorsprung 22 der Feder 17 betätigbar. Insbesondere drückt der Behälter 3 in seiner unteren Endlage - also bei gespanntem Zerstäuber 1 bzw. Druckerzeuger 5 - die Feder 17 derart nieder, dass der Vorsprung 22 den Mikroschalter 21 betätigt.

Beim Darstellungsbeispiel verlaufen die Bewegungen bzw. Hübe des Behälters 3 axial bzw. linear. Jedoch kann von der Überwachungseinrichtung 20 alternativ oder zusätzlich auch eine nicht lineare oder nicht axiale Bewegung des Behälters 3 bei anderer Ausgestaltung des Zerstäubers 1 und/oder eine Bewegung eines sonstigen Teils des Zerstäubers 1, insbesondere bei dessen Betätigung, erfassbar sein. Beispielsweise kann die Überwachungseinrichtung 20 alternativ oder zusätzlich gemäß einer nicht dargestellten Ausführungsvariante eine Betätigung des Zerstäubers 1 bzw. einen tatsächlichen Austrag von Fluid 2 durch Messung der Impedanz der Feder 17, die sich in Abhängigkeit von der Spannlage ändert, erfassen.

Die Überwachungseinrichtung 20 erfasst vorzugsweise, wenn der Behälter 3 die Endlage im gespannten Zustand erreicht und/oder wenn er diese während des Zerstäubungsvorgangs verlässt, als Betätigung des Zerstäubers 1, die gezählt wird. Insbesondere weist die Überwachungseinrichtung 20 eine Steuereinheit 23, vorzugsweise einen Microcontroller o.dgl. auf, um das genannte Zählen und/oder sonstige Funktionen der Überwachungseinrichtung 20 zu realisieren. An die Steuereinheit 23 sind die anderen Komponenten der Überwachungseinrichtung 20 angeschlossen.

Alternativ oder zusätzlich kann die Überwachungseinrichtung 20 auch Bewegungen des Behälters 3 oder eines sonstigen Teils des Zerstäubers 1, wie der Feder 17 oder der Halterung 6, erfassen und insbesondere auswerten. Vorzugsweise werden hierbei die Lage bzw. Position, Geschwindigkeit, diesbezügliche Parameter und insbesondere eine Weg-Zeit-Kurve oder dgl. erfasst und ausgewertet.

Zusätzlich erfasst die Überwachungseinrichtung 20 beim Zählen auch den Zeitpunkt der Betätigung, worauf später noch näher eingegangen wird.

Die Hübe des Behälters 3 stellen also einen Zählwert für die Anzahl der Betätigungen des Zerstäubers 1 und damit für die ausgegebene Menge an Fluid 2 dar. Der Zählwert indiziert also den Füllstand des Fluids 2.

Der Zählwert der Betätigungen des Zerstäubers 1, wobei wahlweise die Anzahl der erfolgten Betätigungen und/oder die Anzahl der noch möglichen Betätigungen mit dem aktuellen Behälter 3 anzeigbar und/oder speicherbar ist bzw. sind, ist vorzugsweise manuell oder selbsttätig, insbesondere beim Wechsel des Behälters 3, rücksetzbar. Vorzugsweise erfolgt das Rücksetzen des Zählwerts selbsttätig nach dem Aufsetzen bzw. Aufstecken des Betätigungsteils 15, wobei das Aufsetzen bzw. Aufstecken des Betätigungsteils 15 vorzugsweise mittels eines Kontaktschalters 24 o.dgl. von der Überwachungseinrichtung 20 erfassbar ist.

Beim Darstellungsbeispiel ist der Kontaktschalter 24 mittels eines federvorgespannten Kontaktstifts auslösbar bzw. betätigbar, wobei der Kontaktstift bei aufgesetztem Betätigungsteil 15 vom Innenteil 14 gegen Federkraft niedergedrückt bzw. eingedrückt wird. Diese Ausführungsvariante hat neben der selbsttätigen Rücksetzung des Zählwerts den weiteren Vorteil, dass bei zusammengebautem Zerstäuber 1 ein (erneutes) Betätigen des Kontaktschalters 24 und ein damit verbundenes Rücksetzen des Zählwerts ausgeschlossen sind. Somit ergibt sich eine einfache, für Bedienungsfehler wenig anfällige Bedienung des Zerstäubers 1.

Der Kontaktschalter 24 kann zusätzlich oder alternativ dazu dienen, die Überwachungseinrichtung 20 einzuschalten bzw. zu aktivieren, insbesondere durch Erfassen des erstmaligen Zusammenbaus des Zerstäubers 1.

Alternativ oder zusätzlich zu dem Kontaktschalter 24 kann ein sonstiger Schalter, wie ein induktiver Schalter, kapazitiver Schalter, Reed-Kontakt, Näherungsschalter o.dgl., oder ein sonstiger geeigneter Sensor eingesetzt werden.

Gemäß einer nicht dargestellten Ausführungsvariante ist der Zerstäuber 1 vorzugsweise derart ausgebildet, dass er nur bei ein- bzw. angebauter Überwachungseinrichtung 20 und/oder nur bei eingeschalteter Überwachungseinrichtung 20 auslösbar bzw. betätigbar ist. Dies kann durch eine entsprechende mechanische und/oder elektrische Verbindung oder Kopplung des Zerstäubers 1 mit der Überwachungseinrichtung 20 oder ggf. mit dem die Überwachungseinrichtung 20 enthaltenden Gehäuseteil bzw. Betätigungsteil 15 realisiert sein.

Insbesondere ist der Zerstäuber 1 bei nicht eingeschalteter Überwachungseinrichtung 20, bei fehlender Überwachungseinrichtung 20, bei fehlendem Betätigungsteil 15 und/oder bei fehlendem Behälter 3 gegen Betätigen gesperrt.

Die Überwachungseinrichtung 20 weist vorzugsweise eine insbesondere optische Anzeigeeinrichtung 25 - beim Darstellungsbeispiel ein Display o.dgl. - auf, insbesondere zur Anzeige des Zustands der Überwachungseinrichtung 20, der vergangenen Zeit nach der letzten Betätigung des Zerstäubers 1, der verbleibenden Zeit bis zur nächsten Betätigung des Zerstäubers 1, der Anzahl der bereits erfolgten Betätigungen des Zerstäubers 1, der Anzahl der noch möglichen Betätigungen des Zerstäubers 1, von noch auszuführenden Betätigungen des Zerstäubers 1 (beispielsweise beim Einsetzen eines neuen Behälters 3), eines erfolgten oder eines erforderlichen Behälterwechsels, des Fluidfüllstands, einer Behälteridentifikation und/oder einer Fluidbezeichnung. Dies gestattet eine optimale Information und ggf. Führung des Benutzers. Somit wird die Handhabung des Zerstäubers 1 erleichtert und eine höhere Bedienungssicherheit ermöglicht.

Die Überwachungseinrichtung 20 weist vorzugsweise eine akustische Anzeigeeinrichtung 26, insbesondere einen Piezosignalgeber o.dgl., auf, insbesondere zur Ausgabe eines Erinnerungssignals zur Erinnerung an eine anstehende Betätigung des Zerstäubers 1 und/oder Anzeige eines andauernden und/oder abgeschlossenen Zerstäubungsvorgangs.

Beispielsweise kann die Überwachungseinrichtung 20 durch Ausgabe eines während des Zerstäubungsvorgangs andauernden Tonsignals dem Benutzer anzeigen, dass eine Zerstäubung erfolgt und der Benutzer das erzeugte Aerosol entsprechend inhalieren soll. Vorzugsweise endet das Tonsignals nicht mit Beendigung des Zerstäubungsvorgangs, sondern erst nach einer darüber hinaus gehenden Zeitdauer, um sicherzustellen, dass der Benutzer das erzeugte Aerosol auch vollständig inhaliert. Aufgrund der verhältnismäßig genau festgelegten Zeitdauer von etwa ein bis zwei Sekunden des Zerstäubungsvorgangs kann die Gesamtzeitdauer des genannten Tonsignals unabhängig vom tatsächlichen Zerstäubungsvorgang durch eine vorzugsweise vorgebbare Zeitdauer, von beispielsweise drei bis vier Sekunden oder von etwa 10 bis 15 Sekunden bei Berücksichtigung eines nach dem Inhalieren erwünschten Luftanhaltens festgelegt werden. Die Überwachungseinrichtung 20 gibt dann das Tonsignal nach Auslösen des Zerstäubungsvorgangs durch Betätigen des Sperrelements 8 - also beginnend mit Detektion des Hubs des Behälters 3 in Zerstäubungsrichtung mittels des Mikroschalters 21 - aus.

Alternativ oder zusätzlich zu dem vorgenannten Tonsignal, das fortlaufend oder wiederholend während des tatsächlichen oder anzuzeigenden Zerstäubungsvorgangs ausgeben wird, kann die Überwachungseinrichtung 20 auch ein Endsignal zur Anzeige des Abschlusses des Zerstäubungsvorgangs, beispielsweise nach Ablauf der vorbestimmten Zeitdauer, ausgeben.

Zusätzlich oder alternativ kann insbesondere mittels der akustischen Anzeigeeinrichtung 26 - ggf. mit einem anderen Signal - einem Benutzer die tatsächliche Dauer des Inhalierens und/oder ein ausreichend starkes Inhalieren und/oder ein nicht ausreichend starkes oder nicht ausreichend langes Inhalieren angezeigt werden.

Vorzugsweise erzeugt die akustische Anzeigeeinrichtung 26 ein Schallsignal, das bedarfsweise durch eine im Betätigungsteil 15 vorgesehene Öffnung austreten kann.

Zusätzlich oder alternativ kann die akustische Anzeigeeinrichtung 26 als Signal ein Vibrieren des Betätigungsteils 15 bzw. des Zerstäubers 1 bewirken, also ein Vibrationssignal oder sonstiges taktiles Signal ausgeben.

Die Überwachungseinrichtung 20 weist einen Energiespeicher, insbesondere eine Batterie 27 oder ggf. einen nicht dargestellten Akkumulator, auf. Der Energiespeicher wird vorzugsweise erst bei Einschalten der Überwachungseinrichtung 20 angeschlossen, um eine lange Lagerfähigkeit bei allenfalls minimalem Energieverlust zu ermöglichen.

Vorzugsweise weist der Energiespeicher bzw. die Batterie 27 eine derartige Kapazität auf, dass die Überwachungseinrichtung 20 nach dem Einschalten mindestens ein Jahr, vorzugsweise mindestens zwei Jahre, insbesondere mindestens fünf Jahre, funktionsfähig bleibt.

Die Überwachungseinrichtung 20 weist ferner eine vorzugsweise nur optisch arbeitende Schnittstelle 28 - beim Darstellungsbeispiel eine Leuchtdiode - auf, die insbesondere ein Einschalten, Programmieren, Einstellen, Rücksetzen und/oder Abfragen der Überwachungseinrichtung 20 ermöglicht.

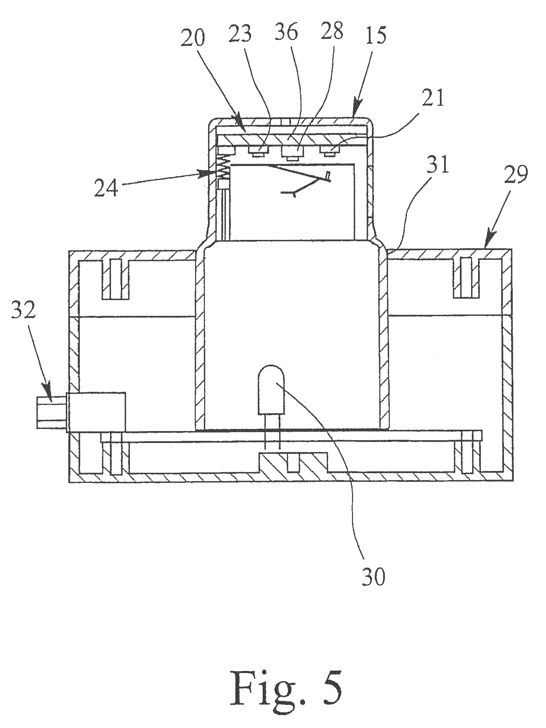

Zur Kommunikation mit der Überwachungseinrichtung 20 über die Schnittstelle 28 ist vorzugsweise eine beispielhaft in Fig. 5 dargestellte Anschlusseinrichtung 29 vorgesehen. Insbesondere ist die Überwachungseinrichtung 20 über die Schnittstelle 28 - ggf. erst nach Trennen der Überwachungseinrichtung 20 zusammen mit dem Gehäuse- bzw. Betätigungsteil 15 vom Zerstäuber 1 - an die Anschlusseinrichtung 29 anschließbar. Vorzugsweise weist die Anschlusseinrichtung 29 zur bevorzugten optischen Kommunikation bzw. Datenübertragung mit der Überwachungseinrichtung 20 eine Leuchtdiode 30 o.dgl. auf.

Zum Anschluss ist die Überwachungseinrichtung 20 bzw. das Betätigungsteil 15 insbesondere in eine entsprechende Aussparung 31 der Anschlusseinrichtung 29 einsetzbar und/oder die Anschlusseinrichtung 29 zumindest teilweise in das Betätigungsteil 15 einführbar.

Die Anschlusseinrichtung 29 ist vorzugsweise an einen nicht dargestellten Computer o. dgl. anschließbar, beispielsweise über einen Anschluss 32. Entsprechend ist die Überwachungseinrichtung 20 sehr einfach initialisierbar, einschaltbar, programmierbar, rücksetzbar, abfragbar o.dgl. Von der Überwachungseinrichtung 20 gespeicherte Daten sind entsprechend einfach abrufbar, anzeigbar und ggf. auswertbar. Dies ist insbesondere für klinische Untersuchungen und/oder eine Patientenüberwachung durch einen Arzt vorteilhaft.

Alternativ oder zusätzlich kann die Anschlusseinrichtung 29 auch unabhängig von einem Computer oder dgl. einsetzbar sein.

Bedarfsweise kann die Anschlusseinrichtung 29 auch ein eigenes Display und/oder eine Tastatur oder sonstige Eingabeeinrichtung aufweisen, um die Überwachungseinrichtung 20 einschalten, programmieren, einstellen, rücksetzen und/oder abfragen zu können. Insbesondere sind dann verschiedene Parameter oder dgl. anzeigbar, wobei in diesem Fall bedarfsweise die optische Anzeigeeinrichtung 25 und/oder eine Eingabeeinrichtung 35 der Überwachungseinrichtung 20 entfallen kann bzw. können.

Statt der Batterie 27 kann bedarfsweise auch ein Akkumulator eingesetzt werden. Ein Aufladen kann dann beispielsweise über einen elektrischen Anschluss oder ggf. induktiv erfolgen, insbesondere gleichzeitig mit dem Anschluss an die Anschlusseinrichtung 29 und/oder über eine nicht dargestellte Solarzelle der Überwachungseinrichtung 20.

Die Überwachungseinrichtung 20 ist vorzugsweise derart ausgebildet, dass sie erst beim erstmaligen Zusammenbau mit dem Zerstäuber 1, beim erstmaligen Einsetzen des Behälters 3, beim erstmaligen Spannen oder Betätigen des Druckerzeugers 5 und/oder beim Initialisieren über die Schnittstelle 28 eingeschaltet wird. Dies ist im Hinblick auf eine lange Lagerfähigkeit vorteilhaft.

Vorzugsweise ist die Überwachungseinrichtung 20 nach dem Einschalten nicht mehr abschaltbar. Dies gewährleistet die gewünschte fortlaufende Überwachung.

Die Überwachungseinrichtung 20 weist vorzugsweise einen Zeitgeber 33 und einen Speicher 34 auf, insbesondere so dass Anzahl und Zeitpunkt der Betätigungen des Zerstäubers 1 bzw. des Austrags von Fluid 2 erfassbar und speicherbar sind und/oder so dass eine wiederholte Betätigung des Zerstäubers 1 innerhalb einer vorbestimmten Mindestzeitdauer blockierbar ist und/oder so dass nach Ablauf einer vorbestimmten Höchstzeitdauer das bereits genannte Erinnerungssignal zur erneuten Betätigung des Zerstäubers 1 ausgebbar ist.

Wenn die Anzahl und Zeitpunkte der Betätigungen bzw. des Austrags von Fluid 2 fortlaufend im Speicher 34 gespeichert werden, kann durch Abfrage der Überwachungseinrichtung 20 - also Auslesen des Speichers 34 - festgestellt und überwacht werden, wann und in welchem Umfang der Zerstäuber 1 benutzt bzw. Fluid 2 ausgegeben worden ist. Die Überwachungsmöglichkeit ist für den Benutzer zur Selbstkontrolle und/oder für eine Überwachung durch den behandelnden Arzt und/oder für klinische Studien - insbesondere zur Überwachung der Einhaltung von verordneten Dosierungen des Fluids 2 - vorteilhaft.

Wenn eine wiederholte Betätigung des Zerstäubers 1 innerhalb einer vorbestimmten Mindestzeitdauer blockierbar ist, kann eine Überdosierung vermieden werden.

Der Speicher 34 weist vorzugsweise einen EPROM oder EEPROM auf, wobei im letzteren Fall ein elektrisches Rücksetzen möglich ist.

Der Speicher 34 bzw. dessen Inhalt ist vorzugsweise zumindest für den Benutzer bzw. Patienten nicht löschbar oder veränderbar.

Vorzugsweise stellt der Zeitgeber 33 keine absolute Zeitbasis dar; vielmehr handelt es sich hierbei um einen (einfachen) Zähler, der nur eine relative Zeit erfasst bzw. zur Verfügung stellt. Dies ermöglicht einen besonders einfachen und kostengünstigen Aufbau.

Der absolute Beginn der relativen Zeiterfassung durch den Zeitgeber 33 wird vorzugsweise beim Initialisieren oder erstmaligen Einschalten der Überwachungseinrichtung 20 festgelegt und ist insbesondere im Speicher 34 oder von einer sonstigen Einrichtung, wie dem nicht dargestellten Computer zur Initialisierung der Überwachungseinrichtung 20, gespeichert.

Alternativ oder zusätzlich kann auch das absolute Ende der relativen Zeiterfassung durch den Zeitgeber 33 einfach beim Abfragen der Überwachungseinrichtung 20 durch Vergleich mit einer absoluten Zeitbasis festgelegt werden. So können die absoluten Zeiten der von der Überwachungseinrichtung 20 erfassten und im Speicher 34 gespeicherten Betätigungen des Zerstäubers 1 festgelegt bzw. festgestellt werden.

Gemäß einer bevorzugten Ausführungsvariante ist bzw. sind der Zerstäuber 1 und/oder die Überwachungseinrichtung 20 derart ausgebildet, dass der eingesetzte Behälter 3 und/oder dessen Füllgrad und/oder dessen Fluid 2 von der Überwachungseinrichtung 20 vorzugsweise selbsttätig identifizierbar und die Behälteridentifizierung insbesondere abspeicherbar und/oder anzeigbar ist. So ist nachvollziehbar, welcher Behälter 3 und damit welches Fluid 2 verwendet worden sind.

Beispielsweise kann die Überwachungseinrichtung 20 bzw. der Zerstäuber 1 einen nicht dargestellten Barcode-Leser oder eine sonstige Einrichtung zur Abfrage einer Identifizierung bzw. Kodierung, wie eines Barcodes, des Behälters 3 aufweisen. So ist beispielsweise feststellbar, ob ein falscher Behälter 3 oder ein Behälter 3 mit einem falschen Fluid 2, einer falschen Fluidmenge und/oder einer falschen Wirkstoffkonzentration des Fluids 2 eingesetzt wird. Je nach Programmierung bzw. Einstellung der Überwachungseinrichtung 20 kann dann eine Betätigung des Zerstäubers 1 auch sperrbar sein.

Vorzugsweise weist die Überwachungseinrichtung 20 eine abfragbare Identifikation zur Identifizierung der Überwachungseinrichtung 20, des Zerstäubers 1 und/oder eines Benutzers auf.

Die Überwachungseinrichtung 20 weist vorzugsweise alternativ oder zusätzlich zur Schnittstelle 28 eine manuell betätigbare Eingabeeinrichtung 35, insbesondere einen Taster, eine Tastatur o. dgl., auf. Vorzugsweise ist mittels der Eingabeeinrichtung 35 die Überwachungseinrichtung 20 einschaltbar, programmierbar, einstellbar, rücksetzbar und/oder abfragbar. Beispielsweise können mittels der Eingabeeinrichtung 35 die Mindestzeitdauer, die Höchstzeitdauer und/oder die Anzahl der Dosierung, beispielsweise die Anzahl der Hübe pro Anwendung und die Anzahl der Anwendungen pro Tag, eingestellt werden.

Alle oder zumindest die meisten Komponenten der Überwachungseinrichtung 20 sind vorzugsweise auf einer Leiterplatte 36 angeordnet und/oder an diese angeschlossen. Insbesondere bildet die Überwachungseinrichtung 20 eine Baugruppe, die in das Gehäuse- bzw. Betätigungsteil 15 des Zerstäubers 1 eingesetzt, vorzugsweise eingegossen, ist.

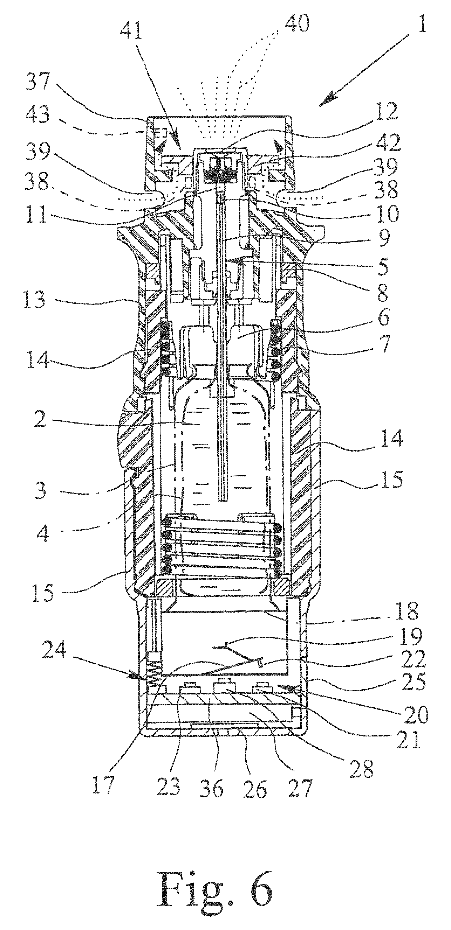

Gemäß einem weiteren, bedarfsweise auch unabhängig realisierbaren Aspekt weist der vorschlagsgemäße Zerstäuber 1 insbesondere im Bereich eines Mundstücks 37 bzw. der Austragsdüse 12 einen Aufnahmesensor 38 zur Erfassung eines Luftstroms und/oder eines Inhalierens von zerstäubtem Fluid 2 auf, wie in Fig. 6 angedeutet.

Vorzugsweise ist der Aufnahmesensor 38 einer Zuluftöffnung 39 zugeordnet, über die insbesondere seitlich oder im Bereich der Austragsdüse 12 Zuluft von einem Benutzer beim Inhalieren ansaugbar ist, wie durch Pfeile in Fig. 6 angedeutet.

Der Aufnahmesensor 38 ist gemäß einer ersten Ausführungsvariante vorzugsweise unmittelbar zur Erfassung eines entsprechenden Zuluftstroms ausgebildet, so dass ein Inhalieren des in Fig. 6 schematisch angedeuteten Aerosols 40 detektiert werden kann.

Vorzugsweise kann der Aufnahmesensor 38 die Richtung eines Luftstroms durch die Zuluftöffnung 39, die Strömungsgeschwindigkeit und/oder den Volumenstrom erfassen. Hierzu kann der Aufnahmesensor 38 beispielsweise als sogenannter Strömungssensor zur unmittelbaren Erfassung einer Luftströmung ausgebildet oder beispielsweise einer nicht dargestellten, vorzugsweise frei schwenkbaren Klappe, einem vom Luftstrom drehbaren Flügelrad oder dgl. zugeordnet sein.

Gemäß einer besonders bevorzugten, bedarfsweise auch unabhängig realisierbaren zweiten Ausführungsvariante ist der Zuluftöffnung 39 bzw. den Zuluftöffnungen 39 ein in Fig. 6 schematisch angedeutetes, insbesondere als Einweg- oder Rückschlagventil ausgebildetes Ventil 41 vorzugsweise mit einem beweglichen Ventilelement 42 zugeordnet, um ein (ungewolltes) Ausblasen des Aerosols 40 durch die Zuluftöffnung(en) 39 verhindern zu können. In diesem Fall erfasst der Aufnahmesensor 38 vorzugsweise unmittelbar nur das Öffnen und/oder Schließen des Ventils 41, also den Zuluftstrom nur indirekt. Dies ermöglicht einen besonders einfachen und kostengünstigen Aufbau, insbesondere wenn der Aufnahmesensor 38 beispielsweise einen nicht dargestellten Mikroschalter aufweist, der vom Ventilelement 42 betätigbar ist.

Der Aufnahmesensor 38 ist vorzugsweise an die Überwachungseinrichtung 20 angeschlossen, die die Signale des Aufnahmesensors 38 entsprechend auswertet und insbesondere speichert. Dies kann bedarfsweise drahtlos oder sogar mechanisch erfolgen. Vorzugsweise ist der Aufnahmesensor 38 elektrisch über nicht dargestellte Leitungen und geeignete Kontakte am Übergang zum Betätigungsteil 15 an die Überwachungseinrichtung 20 angeschlossen.

Mittels des Aufnahmesensors 38 ist der Zuluftstrom und damit ein tatsächliches Inhalieren des mit dem Zerstäuber 1 erzeugten Aerosols 40 erfassbar. Dementsprechend kann die Überwachungseinrichtung 20 alternativ oder zusätzlich zu der Erfassung der Bewegung des Behälters 3 ein mittels des Aufnahmesensors 38 erfasstes Inhalieren des Aerosols 40 als tatsächlichen Austrag oder Aufnahme des Fluids 2 bzw. (erfolgreiche) Betätigung des Zerstäubers 1 zählen und diesen Zählwert im bereits beschriebenen Sinne verarbeiten, anzeigen, speichern o. dgl.

Die mittels des Aufnahmesensors 38 mögliche Erfassung einer tatsächlichen Inhalation des Fluids 2 kann von der Überwachungseinrichtung 20 dahingehend ausgewertet werden, ob ein ausreichend langes Inhalieren erfolgt, wobei die Inhalationszeit bedarfsweise gespeichert und/oder angezeigt werden kann.

Gemäß einer weiteren Ausführungsvariante weist der Zerstäuber 1 zusätzlich oder alternativ zum Aufnahmesensor 38 einen Spraysensor 43 auf, der eine Erfassung ermöglicht, ob tatsächlich eine Zerstäubung erfolgt. Insbesondere erfasst der Spraysensor, ob sich tatsächlich Tröpfchen des zerstäubten Fluids 2 bzw. Aerosol 40 im Bereich des Mundstücks 37 bilden bzw. bildet. Beispielsweise nutzt der Sensor den Effekt, dass das zerstäubte Fluid 2 bzw. Aerosol 40 Licht streut, und arbeitet als sogenannter Streulicht-Sensor.

Mittels des Spraysensors 43 ist also erfassbar, ob tatsächlich eine Zerstäubung erfolgt. Insbesondere überprüft die Überwachungseinrichtung 20, ob bei Betätigung des Zerstäubers 1 - vorzugsweise innerhalb eines vorbestimmten Zeitfensters - tatsächlich auch eine Zerstäubung mittels des genannten Sensors erfasst wird, wobei nur bei Erfassung einer tatsächlichen Zerstäubung das Betätigen des Zerstäubers 1 tatsächlich als Austrag von Fluid 2 und damit als Zerstäubungsvorgang erfasst bzw. gezählt wird.

Alternativ oder zusätzlich zur Erfassung einer Bewegung des Behälters 3 oder eines sonstigen Teils kann die Überwachungseinrichtung 20 mittels des Aufnahmesensors 38 einen Zuluftstrom und/oder mittels des Spraysensors 43 eine Erzeugung von Aerosol 40 detektieren und dies - ggf. auch nur bei kumulativem Auftreten - als tatsächlichen Austrag von Fluid 2 auswerten und als Betätigung des Zerstäubers 1 zählen und ggf. - insbesondere zusammen mit dem jeweiligen Zeitpunkt - speichern.

Weitere Aspekte der vorliegenden Erfindung sind:
1. Zerstäuber (1) für ein Fluid (2), mit einem vorzugsweise einsetzbaren und ggf. wechselbaren Behälter (3) mit dem Fluid (2), mit einem Druckerzeuger (5) zur Förderung und/oder Zerstäubung des Fluids (2) und mit einer Überwachungseinrichtung (20) zur Zählung von Betätigungen des Zerstäubers (1), dadurch gekennzeichnet,
   dass die Überwachungseinrichtung (20) an einem lösbaren Gehäuseteil des Zerstäubers (1) angeordnet und/oder derart ausgebildet ist, dass ein tatsächlicher Austrag von Fluid (2) erfassbar und insbesondere elektronisch als Betätigung des Zerstäubers (1) zählbar ist, und/oder
   dass die Überwachungseinrichtung (20) einen Zeitgeber (33) und einen Speicher (34) aufweist, insbesondere so dass Anzahl und Zeitpunkt der Betätigungen des Zerstäubers (1) erfassbar und speicherbar sind und/oder so dass eine wiederholte Betätigung des Zerstäubers (1) innerhalb einer vorbestimmten Mindestzeitdauer blockierbar ist und/oder so dass vorzugsweise nach Ablauf einer vorbestimmten Höchstzeitdauer ein Erinnerungssignal vorzugsweise zur erneuten Betätigung des Zerstäubers (1) ausgebbar oder anzeigbar ist.
2. Zerstäuber nach Aspekt 1, dadurch gekennzeichnet, dass der Zerstäuber (1) ein insbesondere zum Einsetzen des Behälters (3) lösbares und ggf. auswechselbares Betätigungsteil (15) aufweist.
3. Zerstäuber nach Aspekt 2, dadurch gekennzeichnet, dass das Betätigungsteil (15) zum Betätigen, insbesondere Spannen, des Druckerzeugers (5) drehbar ist.
4. Zerstäuber nach Aspekt 2 oder 3, dadurch gekennzeichnet, dass das Betätigungsteil (15) ein vorzugsweise kappenartiges Außengehäuseteil des Zerstäubers (1) bildet und/oder den Behälter (3) zumindest partiell unmittelbar abdeckt.
5. Zerstäuber nach einem der Aspekte 2 bis 4, dadurch gekennzeichnet, dass der Behälter (3) bei Betätigung des Betätigungsteils (15) in dieses hinein bewegbar ist.
6. Zerstäuber nach einem der Aspekte 2 bis 5, dadurch gekennzeichnet, dass die Überwachungseinrichtung (20) am Betätigungsteil (15) angeordnet, insbesondere darin eingebaut, ist.
7. Zerstäuber nach Aspekt 6, dadurch gekennzeichnet, dass die Überwachungseinrichtung (20) in einem axialen bzw. stirnseitigen Endbereich des Betätigungsteils (15) angeordnet ist.
8. Zerstäuber nach einem der voranstehenden Aspekte, dadurch gekennzeichnet, dass die Überwachungseinrichtung (20) benachbart zu einem Endbereich des Behälters (3), insbesondere einem Behälterboden (18), und/oder in Verlängerung einer Bewegungsrichtung des Behälters (3) angeordnet ist.
9. Zerstäuber nach einem der voranstehenden Aspekte, dadurch gekennzeichnet, dass der Zerstäuber (1) nur bei an- oder eingebauter Überwachungseinrichtung (20) und/oder nur bei eingeschalteter Überwachungseinrichtung (20) betätigbar ist.
10. Zerstäuber nach einem der voranstehenden Aspekte, dadurch gekennzeichnet, dass der Behälter (3) bei Betätigung des Zerstäubers (1) insbesondere axial bewegbar ist und dass die Überwachungseinrichtung (20) unmittelbar eine Bewegung des Behälters (3) erfasst und als Austrag von Fluid (2) auswertet und ggf. zählt.
11. Zerstäuber nach Aspekt 10, dadurch gekennzeichnet, dass die Überwachungseinrichtung (20) Bewegungen des Behälters (3) mechanisch, optisch, elektrisch, induktiv, kapazitiv und/oder anderweit berührungslos erfasst.
12. Zerstäuber nach Aspekt 10 oder 11, dadurch gekennzeichnet, dass die Überwachungseinrichtung (20) einen Mikroschalter (21) oder sonstigen Schalter oder Sensor zur Erfassung von Bewegungen des Behälters (3) aufweist.
13. Zerstäuber nach Aspekt 12, dadurch gekennzeichnet, dass der Mikroschalter (21) oder sonstige Schalter bzw. Sensor vom Behälter (3) über eine Feder (17) betätigbar ist.
14. Zerstäuber nach einem der Aspekte 10 bis 13, dadurch gekennzeichnet, dass die Überwachungseinrichtung (20) zur Erfassung und vorzugsweise Auswertung von Bewegungen des Behälters (3) bei einem Spannen oder Betätigen des Druckerzeugers (5) und/oder bei der Förderung bzw. Zerstäubung des Fluids (2) ausgebildet ist.
15. Zerstäuber nach einem der Aspekte 10 bis 14, dadurch gekennzeichnet, dass die Überwachungseinrichtung (20) ein Erreichen und/oder Verlassen einer Endlage des Behälters (3), vorzugsweise einer Endlage bei gespanntem Druckerzeuger (5), erfasst.
16. Zerstäuber nach einem der voranstehenden Aspekte, dadurch gekennzeichnet, dass die Überwachungseinrichtung (20) die, insbesondere mit dem aktuellen Behälter (3), bereits erfolgten und/oder noch ausführbaren Betätigungen des Zerstäubers (1) zählt und vorzugsweise anzeigt, insbesondere wobei der Zählwert manuell oder selbsttätig beim Wechsel des Behälters (3), insbesondere mittels eines Kontaktschalters (24), rücksetzbar ist, insbesondere wobei das Rücksetzen bei zusammengebautem Zerstäuber (1) gesperrt ist.
17. Zerstäuber nach einem der voranstehenden Aspekte, dadurch gekennzeichnet, dass die Überwachungseinrichtung (20) eine vorzugsweise optische Anzeigeeinrichtung (25) aufweist, insbesondere zur Anzeige der vergangenen Zeit nach der letzten Betätigung des Zerstäubers (1), der verbleibenden Zeit bis zur nächsten Betätigung des Zerstäubers (1), der Anzahl der bereits erfolgten Betätigungen des Zerstäubers (1), der Anzahl der noch möglichen Betätigungen des Zerstäubers (1), von noch auszuführenden Betätigungen des Zerstäubers (1), eines erfolgten oder erforderlichen Behälterwechsels, des Fluidfüllstands, einer Behälteridentifikation und/oder einer Fluidbezeichnung.
18. Zerstäuber nach einem der voranstehenden Aspekte, dadurch gekennzeichnet, dass die Überwachungseinrichtung (20) eine akustische Anzeigeeinrichtung (26), vorzugsweise einen Piezosignalgeber, aufweist, insbesondere zur Ausgabe eines Erinnerungssignals zur Betätigung des Zerstäubers (1) und/oder zur Anzeige eines andauernden und/oder abgeschlossenen Zerstäubungsvorgangs.
19. Zerstäuber nach einem der voranstehenden Aspekte, dadurch gekennzeichnet, dass die Überwachungseinrichtung (20) einen Energiespeicher, insbesondere eine Batterie (27), aufweist, der vorzugsweise erst bei Einschalten der Überwachungseinrichtung (20) angeschlossen wird.
20. Zerstäuber nach Aspekt 19, dadurch gekennzeichnet, dass die Batterie (27) eine derartige Kapazität aufweist, dass die Überwachungseinrichtung (20) nach dem Einschalten mindestens ein Jahr, vorzugsweise mindestens zwei Jahre, insbesondere mindestens fünf Jahre, funktionsfähig bleibt.
21. Zerstäuber nach einem der voranstehenden Aspekte, dadurch gekennzeichnet, dass die Überwachungseinrichtung (20) eine vorzugsweise nur optische Schnittstelle (28), vorzugsweise eine Leuchtdiode, insbesondere zum Einschalten, Initialisieren, Programmieren, Einstellen, Rücksetzen und/oder Abfragen der Überwachungseinrichtung (20), aufweist.
22. Zerstäuber nach einem der voranstehenden Aspekte, dadurch gekennzeichnet, dass die Überwachungseinrichtung (20) derart ausgebildet ist, dass sie erst beim Zusammenbau mit dem Zerstäuber (1), beim erstmaligen Einsetzen des Behälters (3), beim erstmaligen Spannen oder Betätigen des Druckerzeugers (5) und/oder Initialisieren über eine Schnittstelle (28) eingeschaltet wird.
23. Zerstäuber nach einem der voranstehenden Aspekte, dadurch gekennzeichnet, dass die Überwachungseinrichtung (20) derart ausgebildet ist, dass sie nach dem Einschalten nicht mehr abschaltbar ist.
24. Zerstäuber nach einem der voranstehenden Aspekte, dadurch gekennzeichnet, dass mit dem Zeitgeber (33) nur eine relative Zeit erfassbar ist.
25. Zerstäuber nach Aspekt 24, dadurch gekennzeichnet, dass der absolute Beginn der relativen Zeiterfassung durch den Zeitgeber (33) beim Initialisieren oder erstmaligen Einschalten der Überwachungseinrichtung (20), insbesondere im Speicher (34), speicherbar oder festlegbar ist.
26. Zerstäuber nach Aspekt 24 oder 25, dadurch gekennzeichnet, dass das absolute Ende der relativen Zeiterfassung durch den Zeitgeber (33) beim Abfragen der Überwachungseinrichtung (20) durch Vergleich mit einer absoluten Zeitbasis feststellbar ist.
27. Zerstäuber nach einem der Aspekte 24 bis 26, dadurch gekennzeichnet, dass der Speicher (34), zumindest für einen Benutzer des Zerstäubers (1), nicht löschbar oder veränderbar ist.
28. Zerstäuber nach einem der voranstehenden Aspekte, dadurch gekennzeichnet, dass die Überwachungseinrichtung (20) eine abfragbare Identifikation zur Identifizierung der Überwachungseinrichtung (20), des Zerstäubers (1) und/oder eines Benutzers aufweist.
29. Zerstäuber nach einem der voranstehenden Aspekte, dadurch gekennzeichnet, dass der Zerstäuber (1) und/oder die Überwachungseinrichtung (20) derart ausgebildet ist bzw. sind, dass der eingesetzte Behälter (3) von der Überwachungseinrichtung (20) vorzugsweise selbsttätig identifizierbar ist und die Behälteridentifizierung insbesondere abspeicherbar ist.
30. Zerstäuber nach einem der voranstehenden Aspekte, dadurch gekennzeichnet, dass die Überwachungseinrichtung (20) eine Eingabeeinrichtung (35), insbesondere zum Einschalten, Initialisieren, Programmieren, Einstellen, Rücksetzen und/oder Abfragen der Überwachungseinrichtung (20), aufweist.
31. Zerstäuber nach einem der voranstehenden Aspekte, dadurch gekennzeichnet, dass der Zerstäuber (1) einen Aufnahmesensor (38) zur Erfassung eines Zuluftstroms insbesondere im Bereich eines Mundstücks (37) aufweist, wobei Signale des Aufnahmesensors (38) von der Überwachungseinrichtung (20) erfassbar sind.
32. Zerstäuber nach Aspekt 31, dadurch gekennzeichnet, dass der Zuluftstrom von einem Benutzer des Zerstäubers (1) beim Inhalieren erzeugbar ist.
33. Zerstäuber nach Aspekt 31 oder 32, dadurch gekennzeichnet, dass die Überwachungseinrichtung (20) die Signale des Aufnahmesensors (38) hinsichtlich eines ausreichend starken und/oder ausreichend langen Inhalierens, insbesondere während des jeweiligen Zerstäubungsvorgangs und/oder nach jedem Zerstäubungsvorgang, auswertet, speichert, anzeigt und/oder als erfolgreiche Inhalation von Fluid (2) bzw. tatsächlichen Austrag von Fluid (2) zählt.
34. Zerstäuber nach einem der Aspekte 31 bis 33, dadurch gekennzeichnet, dass der Aufnahmesensor (38) einer Zuluftöffnung (39) des Zerstäubers (1) zugeordnet ist.
35. Zerstäuber nach Aspekt 34, dadurch gekennzeichnet, dass der Zuluftöffnung (39) ein Ventil (41) zugeordnet ist und der Aufnahmesensor (38) eine Bewegung eines Ventilelements (42) des Ventils (41), vorzugsweise mittels eines Mikroschalters, erfasst.
36. Zerstäuber nach einem der Aspekte 31 bis 35, dadurch gekennzeichnet, dass der Aufnahmesensor (38) als Strömungssensor, insbesondere zur Erfassung einer Strömungsrichtung, der Strömungsgeschwindigkeit, des Volumenstroms und/oder des Massenstroms, ausgebildet ist.
37. Zerstäuber nach einem der voranstehenden Aspekte, dadurch gekennzeichnet, dass der Zerstäuber (1) einen Spraysensor (43) zur Erfassung, ob bei Betätigung des Zerstäubers (1) tatsächlich zerstäubtes Fluid (2) bzw. Aerosol (40), insbesondere im Bereich eines Mundstücks (37), erzeugt bzw. ausgetragen wird, aufweist.
38. Zerstäuber nach Aspekt 37, dadurch gekennzeichnet, dass der Spraysensor (43) als Streulichtsensor arbeitet.
39. Zerstäuber nach Aspekt 37 oder 38, dadurch gekennzeichnet, dass die Überwachungseinrichtung (20) eine vom Spraysensor (43) erfasste Erzeugung von zerstäubtem Fluid (2) bzw. Aerosol (40) als tatsächlichen Austrag von Fluid (2) zählt.
40. Zerstäuber nach Aspekt 39, dadurch gekennzeichnet, dass die Überwachsungseinrichtung (20) nur dann eine Betätigung des Zerstäubers (1) als tatsächlichen Austrag von Fluid (2) zählt, wenn insbesondere innerhalb eines Zeitfensters eine Erzeugung von zerstäubtem Fluid (2) bzw. Aerosol (40) vom Spraysensor (43) erfasst wird.
41. Zerstäuber nach einem der voranstehenden Aspekte, dadurch gekennzeichnet, dass der Druckerzeuger (5) nur mechanisch arbeitet und manuell betätigbar, insbesondere gegen Federkraft spannbar, ist.
42. Zerstäuber nach einem der voranstehenden Aspekte, dadurch gekennzeichnet, dass die Druckerzeugung bzw. Zerstäubung rein mechanisch, insbesondere treibgasfrei, vorzugsweise durch Federkraft, erfolgt.
43. Zerstäuber nach einem der voranstehenden Aspekte, dadurch gekennzeichnet, dass der Zerstäuber (1) als Inhalator, insbesondere zur medizinischen Aerosol-Therapie, ausgebildet ist.

### Bezugszeichenliste

- 1: Zerstäuber
- 2: Fluid
- 3: Behälter
- 4: Beutel
- 5: Druckerzeuger
- 6: Halterung
- 7: Antriebsfeder
- 8: Sperrelement
- 9: Förderrohr
- 10: Rückschlagventil
- 11: Druckkammer
- 12: Austragsdüse
- 13: Gehäuseoberteil
- 14: Innenteil
- 15: Betätigungsteil
- 16: Halteelement
- 17: Feder
- 18: Behälterboden
- 19: Anstechelement
- 20: Überwachungseinrichtung
- 21: Mikroschalter
- 22: Vorsprung
- 23: Steuereinheit
- 24: Kontaktschalter
- 25: Anzeigeeinrichtung optisch
- 26: Anzeigeeinrichtung akustisch
- 27: Batterie
- 28: Schnittstelle
- 29: Anschlusseinrichtung
- 30: Leuchtdiode
- 31: Aussparung
- 32: Anschluss
- 33: Zeitgeber
- 34: Speicher
- 35: Eingabeeinrichtung
- 36: Leiterplatte
- 37: Mundstück
- 38: Aufnahmesensor
- 39: Zuluftöffnung
- 40: Aerosol
- 41: Ventil
- 42: Ventilelement
- 43: Spraysensor

## Patentansprüche

1. Zerstäuber (1) für ein Fluid (2), mit einem vorzugsweise einsetzbaren und ggf. wechselbaren Behälter (3) mit dem Fluid (2), mit einem Druckerzeuger (5) zur Förderung und/oder Zerstäubung des Fluids (2) und mit einer Überwachungseinrichtung (20) zur Zählung und/oder insbesondere elektronischen Erfassung von Betätigungen des Zerstäubers (1), vorzugsweise wobei der Zerstäuber (1) als Inhalator, insbesondere zur medizinischen Aerosol-Therapie, ausgebildet ist,
**dadurch gekennzeichnet,**
**dass** der Behälter (3) bei Betätigung des Zerstäubers (1) insbesondere axial bewegbar ist und dass die Überwachungseinrichtung (20) unmittelbar eine Bewegung des Behälters (3) erfasst; und/oder
**dass** die Überwachungseinrichtung (20) an einem lösbaren Gehäuseteil angeordnet ist und die Überwachungseinrichtung (20) Bewegungen bzw. Hübe des Behälters (3) erfasst; und/oder
**dass** die Überwachungseinrichtung (20) an einem lösbaren Gehäuseteil angeordnet ist und der Zerstäuber (1) ein insbesondere zum Einsetzen des Behälters (3) lösbares und ggf. auswechselbares Betätigungsteil (15) aufweist, wobei der Behälter (3) bei Betätigung des Betätigungsteils (15) in dieses hinein bewegbar ist.

2. Zerstäuber nach Anspruch 1, **dadurch gekennzeichnet, dass** die Überwachungseinrichtung (20) einen Sensor zur Erfassung von Bewegungen des Behälters (3) aufweist.

3. Zerstäuber nach Anspruch 1 oder 2, **dadurch gekennzeichnet, dass** die Überwachungseinrichtung (20) Bewegungen des Behälters (3) optisch, induktiv, kapazitiv und/oder anderweit berührungslos erfasst.

4. Zerstäuber nach einem der voranstehenden Ansprüche, **dadurch gekennzeichnet, dass** der Behälter (3) bei Betätigung des Zerstäubers (1) insbesondere axial bewegbar ist und dass die Überwachungseinrichtung (20) unmittelbar eine Bewegung des Behälters (3) erfasst.

5. Zerstäuber nach einem der voranstehenden Ansprüche, **dadurch gekennzeichnet, dass** die Überwachungseinrichtung (20) ein Erreichen und/oder Verlassen einer Endlage des Behälters (3), vorzugsweise einer Endlage bei gespanntem Druckerzeuger (5), erfasst, vorzugsweise als Betätigung des Zerstäubers (1), die insbesondere gezählt wird.

6. Zerstäuber nach einem der voranstehenden Ansprüche, **dadurch gekennzeichnet, dass** die Überwachungseinrichtung (20) benachbart zu einem Endbereich des Behälters (3), insbesondere einem Behälterboden (18), und/oder in Verlängerung einer Bewegungsrichtung des Behälters (3) angeordnet ist.

7. Zerstäuber nach einem der voranstehenden Ansprüche, **dadurch gekennzeichnet, dass** die Überwachungseinrichtung (20) zur Erfassung und vorzugsweise Auswertung von Bewegungen des Behälters (3) bei einem Spannen oder Betätigen des Druckerzeugers (5) und/oder bei der Förderung bzw. Zerstäubung des Fluids (2) ausgebildet ist.

8. Zerstäuber nach einem der voranstehenden Ansprüche, **dadurch gekennzeichnet, dass** ein Zählwert der Betätigungen des Zerstäubers (1) vorzugsweise manuell oder selbsttätig, insbesondere beim Wechsel des Behälters (3), rücksetzbar ist, vorzugsweise wobei die Anzahl der erfolgten Betätigungen und/oder die Anzahl der noch möglichen Betätigungen mit dem aktuellen Behälter (3) anzeigbar und/oder speicherbar ist bzw. sind.

9. Zerstäuber nach einem der voranstehenden Ansprüche, **dadurch gekennzeichnet, dass** das Betätigungsteil (15) zum Betätigen, insbesondere Spannen, des Druckerzeugers (5) drehbar ist; und/oder
dass das Betätigungsteil (15) ein vorzugsweise kappenartiges Außengehäuseteil des Zerstäubers (1) bildet und/oder den Behälter (3) zumindest partiell unmittelbar abdeckt; und/oder
dass die Überwachungseinrichtung (20) am Betätigungsteil (15) angeordnet, ist, insbesondere wobei die Überwachungseinrichtung (20) in einem axialen bzw. stirnseitigen Endbereich des Betätigungsteils (15) angeordnet ist.

10. Zerstäuber nach einem der voranstehenden Ansprüche, **dadurch gekennzeichnet, dass** die Überwachungseinrichtung (20) eine akustische Anzeigeeinrichtung (26), vorzugsweise einen Piezosignalgeber, aufweist, insbesondere zur Ausgabe eines Erinnerungssignals zur Betätigung des Zerstäubers (1) und/oder zur Anzeige eines andauernden und/oder abgeschlossenen Zerstäubungsvorgangs.

11. Zerstäuber nach einem der voranstehenden Ansprüche, **dadurch gekennzeichnet, dass** die Überwachungseinrichtung (20) einen Energiespeicher, insbesondere eine Batterie (27), aufweist, der vorzugsweise erst bei Einschalten der Überwachungseinrichtung (20) angeschlossen wird, insbesondere wobei die Batterie (27) eine derartige Kapazität aufweist, dass die Überwachungseinrichtung (20) nach dem Einschalten mindestens ein Jahr, vorzugsweise mindestens zwei Jahre, insbesondere mindestens fünf Jahre, funktionsfähig bleibt.

12. Zerstäuber nach einem der voranstehenden Ansprüche, **dadurch gekennzeichnet, dass** die Überwachungseinrichtung (20) derart ausgebildet ist, dass sie nach dem Einschalten nicht mehr abschaltbar ist; und/oder

13. Zerstäuber nach einem der voranstehenden Ansprüche, **dadurch gekennzeichnet, dass** die Überwachungseinrichtung (20) einen Zeitgeber (33) und einen Speicher (34) aufweist, insbesondere so dass Anzahl und Zeitpunkt der Betätigungen des Zerstäubers (1) erfassbar und speicherbar sind und/oder so
dass eine wiederholte Betätigung des Zerstäubers (1) innerhalb einer vorbestimmten Mindestzeitdauer blockierbar ist und/oder so dass vorzugsweise nach Ablauf einer vorbestimmten Höchstzeitdauer ein Erinnerungssignal vorzugsweise zur erneuten Betätigung des Zerstäubers (1) ausgebbar oder anzeigbar ist, vorzugsweise wobei mit dem Zeitgeber (33) nur eine relative Zeit erfassbar ist, insbesondere wobei der absolute Beginn der relativen Zeiterfassung durch den Zeitgeber (33) beim Initialisieren oder erstmaligen Einschalten der Überwachungseinrichtung (20), insbesondere im Speicher (34), speicherbar oder festlegbar ist, insbesondere wobei der Speicher (34), zumindest für einen Benutzer des Zerstäubers (1), nicht löschbar oder veränderbar ist.

14. Zerstäuber nach einem der voranstehenden Ansprüche, **dadurch gekennzeichnet, dass** die Überwachungseinrichtung (20) eine abfragbare Identifikation zur Identifizierung der Überwachungseinrichtung (20), des Zerstäubers (1) und/oder eines Benutzers aufweist.

15. Zerstäuber nach einem der voranstehenden Ansprüche, **dadurch gekennzeichnet, dass** der Druckerzeuger (5) nur mechanisch arbeitet und manuell betätigbar, insbesondere gegen Federkraft spannbar, ist und/oder dass die Druckerzeugung bzw. Zerstäubung rein mechanisch, insbesondere treibgasfrei, vorzugsweise durch Federkraft, erfolgt.
